# EUROPEAN PATENT APPLICATION

(11) **EP 4 252 746 A1**
(43) Date of publication of application: **04.10.2023**
(21) Application number: 22461531.0
(22) Date of filing: 30.03.2022
(51) Int. Cl.: A61K 9/24, A61K 9/16, A61K 9/48, A61K 9/50

(54) **PHARMACEUTICAL COMPOSITION COMPRISING OF RIVAROXABAN AND ACETYLSALICYLIC ACID**

(71) Applicant: PozLab sp. z o.o., 66-775 Poznan (PL)
(72) Inventor: Shlieout, George, 31319 Sehnde (DE); Milewski, Marek, 62-030 Lubo (PL); Ma ycha, Stefan, 63-004 Tulce (PL); Kaczmarek, Mateusz, 63-400 Ostrow Wielkopolski (PL); Breitenbach, Jörg, 68199 Mannheim (DE); Gurynowicz, Ma gorzata, 60-179 Pozna (PL)
(74) Representative: Witek, Rafal

(57) **Abstract**

It has been disclosed a pharmaceutical single unit dosage form comprising of Rivaroxaban and Acetylsalicylic Acid (Aspirin, ASA), in particular for use in the antithrombotic therapy, in particular in treatment or prevention of thromboembolic disorders, atherosclerotic thrombotic events, especially in adult patients after acute coronary syndrome (ACS) with elevated cardiac biomarkers, or other patients with chronic diseases.

## Description

### Field of the Invention

Present invention relates to the field of pharmaceutical composition. Presented invention concerns pharmaceutical composition comprising of Rivaroxaban and Acetylsalicylic Acid (Aspirin, ASA), in particular for use in the antithrombotic therapy, in particular in treatment or prevention of thromboembolic disorders, atherosclerotic thrombotic events, especially in adult patients after acute coronary syndrome (ACS) with elevated cardiac biomarkers, or other patients with chronic diseases.

### Background of the Invention

Rivaroxaban and Aspirin are used for treatment of thromboembolic disorder. Whereas Rivaroxaban is used in doses from 2.5 mg to 20 mg and formulated as a tablet of immediate release characteristics, ASA in doses from 30 mg to 325 mg is mostly formulated as delayed release formulation to protect from high acidic media in the stomach which could cause a risk increase of Gastro-Intestinal-Bleeding (GIB).
Maintenance of normal haemostasis - the balance between bleeding and thrombosis - is a subject to a complex regulatory mechanism. Uncontrolled activation of the coagulant system or defective inhibition of the activation processes may cause formation of local thrombi or embolisms in vessels (arteries, veins) or in heart cavities. This may lead to serious disorders, such as myocardial infarction, angina pectoris (including unstable angina), vascular reocclusions and restenosis after angioplasty or aortocoronary bypass, stroke, transitory ischaemic attacks, peripheral arterial occlusive disorders, pulmonary embolisms or deep vein thromboses; herein below, these disorders are collectively also referred to as thromboembolic disorders. In addition, in the case of consumption coagulopathy, hypercoagulability may - systemically - result in disseminated intravascular coagulation.
The combination of Rivaroxaban and ASA is commonly used to get the balance between bleeding and thrombosis in the clinical practice and they are dosed individually as separate dosage forms.
Rivaroxaban is a selective direct factor Xa inhibitor that is used to prevent and treat venous thromboembolism and to prevent stroke or systemic embolism in atrial fibrillation. Among patients with an acute coronary syndrome, the risk of cardiovascular death, stroke, or myocardial infarction was lower with Rivaroxaban (2.5 mg or 5 mg twice daily) than with placebo, and mortality was lower with the dose of 2.5 mg twice daily than with placebo (Mega JL, Braunwald E, Wiviott SD, et al. Rivaroxaban in patients with a recent acute coronary syndrome. N Engl J Med 2012; 366:9-1). However, the risk of major bleeding was higher with Rivaroxaban (at either dose) than with placebo.
The Cardiovascular Outcomes for People Using Anticoagulation Strategies (COMPASS) trial tested the hypothesis that Rivaroxaban in combination with Aspirin or given alone is more effective than Aspirin alone in preventing recurrent cardiovascular events, with acceptable safety, in patients with stable atherosclerotic vascular disease. The clear result of this study, that the combination reduced strokes, heart attacks and cardiovascular death by practically 25% compared to either drug alone in both patients with stable coronary or peripheral artery disease, was evident (John W. Eikelboom et al., Rivaroxaban with or without Aspirin in Stable Cardiovascular Disease N Engl J Med 2017; 377:1319-1330).
Rivaroxaban and Acetylsalicylic Acid are active pharmaceutical ingredients in medicinal products widely used nowadays as anticoagulants. There is currently one marketed product comprising Rivaroxaban (Xarelto^{®}, Bayer AG) and many products of ASA available on the market. The co-administration of Rivaroxaban and ASA is now one of the standard antithrombotic therapy practices, this co-administration is indicated for the prevention of atherosclerotic thrombotic events in adult patients after acute coronary syndrome (ACS) with elevated cardiac biomarkers. Currently, patients must take two different products, which significantly affects adherence to treatment recommendations and potentially leads to dosing errors Treatment of chronic diseases usually involves long-term use of pharmacotherapy. Although these medications are effective in controlling disease, the full benefits are often not achieved, as about 50% of patients do not take their medications as prescribed. In 2003 report on medication adherence WHO quoted Haynes et al.'s statement that "increasing the effectiveness of adherence interventions can have a much greater impact on population health than any improvement in specific treatments." Among patients with chronic diseases, about 50% do not take their medication as prescribed (Sabaté E, ed. Adherence to Long-Term Therapies: Evidence for Action: World Health Organization; 2003/ Lee JK et al. JAMA. 2006;296(21):2563-2571). This leads to increased morbidity, mortality and incurs costs of approximately $100 billion annually (Osterberg L et al. N Engl J Med. 2005;353(5):487-497).

For example, a meta-analysis comparing therapeutic regimens with FDC-type combination formulations to regimens using combinations of single-component formulations in more than 20000 patients showed a 26% reduction in the risk of nonadherence associated with the introduction of complex type FDC (Bangalore S et al. Am J Med. 2007 Aug; 120(8):713-9). It is expected that the introduction of FDCs will improve the quality of therapy, resulting in a decrease in the incidence of severe conditions and disease complications.

The registered therapeutic indications recommend co-administering a dose of 2.5 mg twice daily of Rivaroxaban with:
1) 75-100 mg/day ASA or ASA plus Clopidogrel for the prevention of thromboembolic complications in patients after an acute coronary incident with elevated cardiac markers
2) 75-100 mg/day ASA as prevention of thromboembolic complications in patients with chronic coronary artery disease (CAD) or symptomatic PAD and a high risk of ischemic events.
Compliance with treatment is a sine qua non for successful treatment of chronic conditions in many fields like hypertension. Fixed-dose combinations are designed to simplify the medication regimen and potentially improve compliance (Sripal Bangalore, Gayathri Kamalakkannan, Sanobar Parkar, Franz H Messerli Fixed-dose combinations improve medication compliance: a meta-analysis, J Med 2007 Aug; 120(8):713-9.)
Up to the present, both active ingredients have been dosed individually and not formulated as a fixed dose combination for obvious reasons which present major technical challenges: Rivaroxaban is unstable in acidic media as reposted in (Bulletin of Faculty of Pharmacy, Cairo University; Volume 53, Issue 1, June 2015, Pages 53-61). The half-life (t_{0.5}) for acid catalysed hydrolysis determined using assays by using HPLC and TLC are 157.5 and 154.0 min. respectively. Aspirin is an acid with its acid dissociation constant (p*K*ₐ) of 3.5 at 25 C. To combine a drug that undergoes acidic hydrolysis with an acidic compound is counterintuitive as it poses the risk of instability and chemical decomposition to the resulting product. Rivaroxaban belongs to the BCS class II (Biopharmaceutical Classification System) with an aqueous solubility of 10 mg/L (K. Takács-Novák, M. Urac, P. Horváth, G. Völgyi, B. D. Anderson, A. Avdeef: Equilibrium solubility measurement of compounds with low dissolution rate by Higuchi's Facilitated Dissolution Method. A validation study. Eur. J. Pharm. Sci.106, 2017, S. 133-144) whereas the acid Aspirin belongs to the BCS I class with a solubility of 3g/L (J. Dressman et al. Biowaiver monograph for immediate-release solid oral dosage forms: acetylsalicylic acid; J Pharm Sci 2012 Aug; 101(8):2653-67.)
In light of the above, the fixed-dose combination design of two compounds belonging to different BCS classes and maintaining the performance of the individual compounds matching the performance of the separately dosed currently marketed products reveals as extremely challenging.
Rivaroxaban is formulated as a solid dosage form - non-functional coated tablet with an immediate release profile, whereas Aspirin for discussed therapeutic indications is formulated as a delayed release formulation (enteric coated) which would release the API only at the pH value ≥ 5.5.
Formulating one dosage form matching two different release performances is the resulting challenge for a fixed dose combination for the two actives.

### The subject of the invention

The aim of subject of the invention is to provide a pharmaceutical composition comprising a combination of Rivaroxaban and Aspirin in the form of one combined drug dosage form, which may be used twice daily in a combined dose: 2.5 mg Rivaroxaban + 50 mg ASA.

The particular object of the invention is to provide such form of the combined drug which maintain gastric resistance effect of ASA tablet at pH 1.2: < 10% after 2h and simultaneously provides ASA dissolution at pH 6.8: > 75% within 90 minutes.

Moreover, the same form of the combined drug has to provide dissolution of Rivaroxaban at pH 4.5 as QC test > 80% within 30 minutes.

### Summary of the Invention

Surprisingly the complex technical effect which has been defined above was achieved by the present invention.

The subject of the invention is pharmaceutical single unit dosage form characterised in that it comprises Acetylsalicylic Acid and Rivaroxaban, wherein said single unit dosage form:
- maintains gastric resistance effect of Acetylsalicylic Acid at pH 1.2: < 10% after 2h and simultaneously provides its dissolution at pH 6.8: > 75% within 90 minutes, and
- provides dissolution of Rivaroxaban at pH 4.5 in QC test > 80% within 30 minutes.

In one aspect the present invention provides a pharmaceutical single dosage unit in form of coated tablet, comprising:
1) a core comprising: Acetylsalicylic Acid, possibly formulated with a pharmaceutically acceptable excipient of a filler/binder function (preferably cellulose and/or its derivatives) and/or a pharmaceutically acceptable excipient of a filler/binder/disintegrant function (preferably starch), possibly in dry weight ratio 5:1:1, respectively,
2) an inner film coating comprising: a pharmaceutically acceptable excipient of a release control polymer, resistant at pH < 5.5 (eg. methacrylic acid and ethylacrylate copolymer, Hydroxypropyl Methyl Cellulose Phthalate, Hydroxypropyl Methyl Cellulose acetate succinate), a pharmaceutically acceptable excipient of a surfactant function (possibly Polysorbate 80, sodium lauryl sulphate), a pharmaceutically acceptable excipient of an anti-tacking function (preferably talc) and a pharmaceutically acceptable excipient of a plasticizer function (preferably triethylcitrate or polyethyleneglycol (PEG)), possibly in dry weight ratio 11.777:0.279:0.085:3.644: 1.215, respectively,
3) an outer film coating consisting of: Rivaroxaban, a pharmaceutically acceptable excipient of a binder/film-forming agent function (preferably HPMC, PVA, HPC) and a pharmaceutically acceptable excipient of a disintegrant function (preferably crospovidone), possibly in dry weight ratio 1:2 :1, respectively,
and optionally a pharmaceutically acceptable excipient of an anti-tacking function, preferably talc.

In next aspect the present invention provides a pharmaceutical single unit dosage in form of capsule, comprising:
1) film coated granules comprising:
   - a core comprising: Acetylsalicylic Acid, possibly formulated with a pharmaceutically acceptable excipient of a filler/binder function (preferably cellulose and/or its derivatives) and/or a pharmaceutically acceptable excipient of a filler/binder/disintegrant function (preferably starch), possibly in dry weight ratio 5:1:1, respectively,
   - a film coating comprising: a pharmaceutically acceptable excipient of a release control polymer, resistant at pH < 5.5 (eg. methacrylic acid and ethyl acrylate copolymer), a pharmaceutically acceptable excipient of a surfactant function (preferably Polysorbate 80 and sodium lauryl sulphate), a pharmaceutically acceptable excipient of an anti-tacking function (preferably talc) and a pharmaceutically acceptable excipient of a plasticizer function (preferably triethyl citrate), preferably in dry weight ratio 11.777 : 0.279 : 0.085 : 3.644 : 1.215, respectively,
2) dry mixture comprising Rivaroxaban formulated with pharmaceutically acceptable excipients, preferably mixture of: PEG, sorbitol, sodium croscarmellose, cellulose, silica, sodium lauryl sulphate and magnesium stearate, possibly in dry weight ratio from 2,5:9,25:1,725:6,9:23,0:1,75:0,088:0,35 to 20:37,0:13,8:27,6:189,374:7,0:0,7, respectively,
   1) hard capsule, possibly comprising gelatine or HPMC.

In next aspect, the present invention provides a pharmaceutical single unit dosage form according to the invention for use in prevention or treatment of thromboembolic disorders, atherosclerotic thrombotic events, especially in adult patients after acute coronary syndrome (ACS) with elevated cardiac biomarkers, or other patients with chronic diseases. Preferably, the pharmaceutical single unit dosage form for use according to the invention is dosed in 12 hour interval.

### Detailed description of the invention and its advantages

In this invention a fixed-dose combination which decreases the risk of medication non-compliance and dosing errors is disclosed for the combination of Rivaroxaban and Aspirin in patients with chronic conditions for improving medication compliance which can translate into better clinical outcomes.

The pharmaceutical composition according to subject invention allows to achieve a number of beneficial effects for the patient.

Many patients benefit from receiving more than one active substance to treat a condition if their effects are synergistic or at least additive. Efficacy can often be significantly improved with minimal impact on the safety and tolerability of the combination product.

Antiplatelet therapy, including ASA, has been shown in many studies to reduce the risk of cardiovascular events and death. The importance of ASA is addressed in the latest 2016 European Society of Cardiology (ESC) guidelines on cardiovascular disease prevention. Most recommendations are Class IA and address the acute phase after acute coronary syndromes, the chronic phase after myocardial infarction, chronic coronary artery disease. ASA in cardiovascular doses for secondary prevention is the absolute canon. It provides the highest risk patients with a 20-30% reduction in the most serious cardiac incidents. However, many patients are known to experience cardiovascular events despite aspirin treatment, which is often referred to as "low responsiveness". Proposed way to eliminate this is to divide the recommended daily dose into 2 servings.

It is easier to remember to take one medication than two. This happens especially when they are dosed in different schedules, such as once and twice a day. Combining them in a single dosage form, adjusting the composition to allow combined dosing makes adherence much easier for patients, especially if they are taking drugs for other indications. This has the effect of improving efficacy by providing more consistent dosing. The results reported in most clinical trials are dependent on consistent dosing. Making it easier to take a prescribed product increases the likelihood that an individual will follow a clinically validated dosing regimen. Combine two active pharmaceutical ingredients in one product and improving compliance is not only easier, but better. Combining two active pharmaceutical ingredients in reduced strengths (in comparison with single preparations) into one dosage unit creates possibility of mass and volume reduction of the final dosage form in comparison with two separately administered units. This leads to improvement in the field of compliance, especially for the elderly patients and those with dysphagia condition.

Furthermore, in comparison with only one marketed product containing Rivaroxaban (Xarelto^{®}, Bayer AG) presented invention has some additional advantages regarding composition as it does not contain or contains in a reduced quantities in comparison to a combination of currently available marketed products:
- lactose or derivatives thereof - disaccharide of dairy origin which is used widely as bulking agent in pharmaceutical products. Lactose consumption may lead to intolerance (of alimentary origin) as well to case of allergy in some group of patients especially if drug is administered in a long-term therapy. This cause significant discomfort and can decrease compliance.
- Ferric Oxide(s) and Titanium Dioxide - inorganic dyes used widely as colorants and opacifiers (especially TiO₂). Recently controversies around them (nanoparticles issue etc.) have been mounting up and until reliable results are not available, those materials should be used with great care.

Therefore, mentioned symptoms and phenomenon will most likely not occur in case of use of presented invention.

The following examples are merely illustrative of the present invention and they should not be considered as limiting the scope of the invention in any way, as these examples, modifications and other equivalents thereof (in particular the dose proportional preparations of the listed formulas) will become apparent to those versed in the art in the light of the present disclosure, and the accompanying claims.

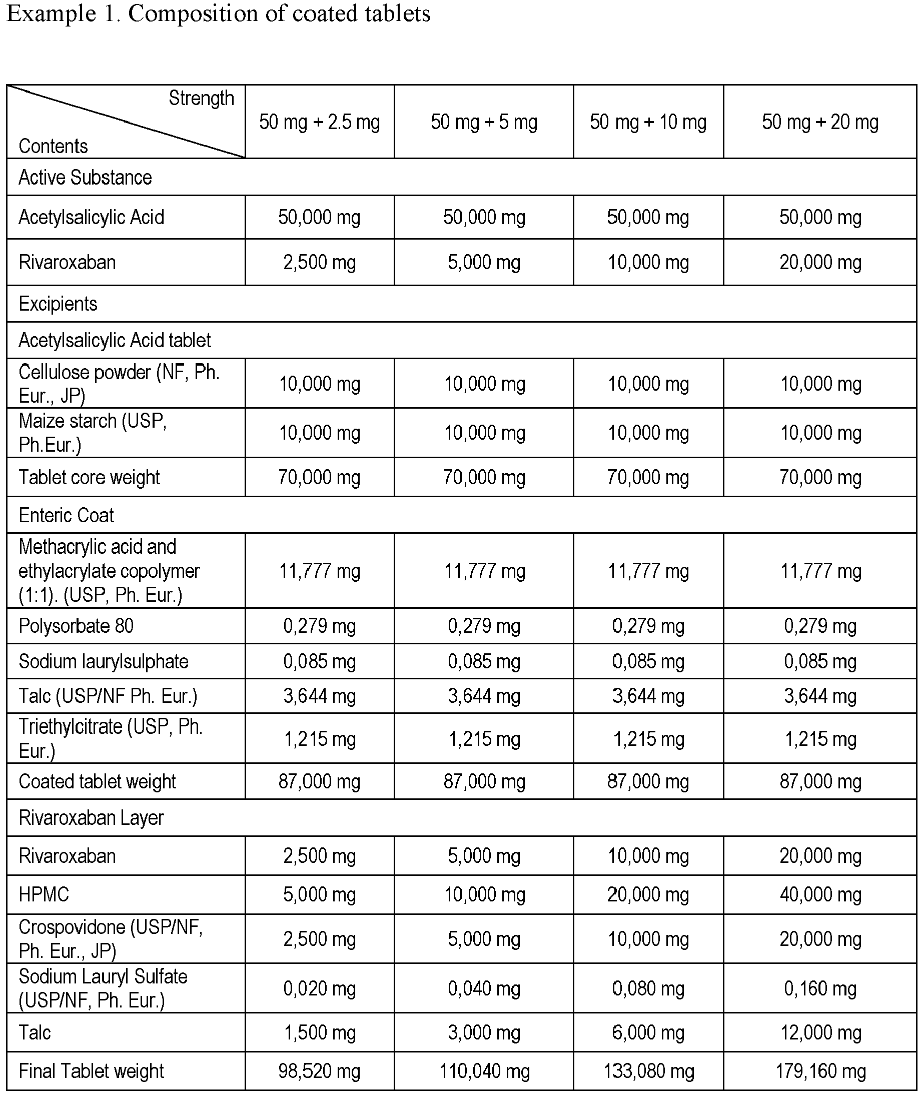

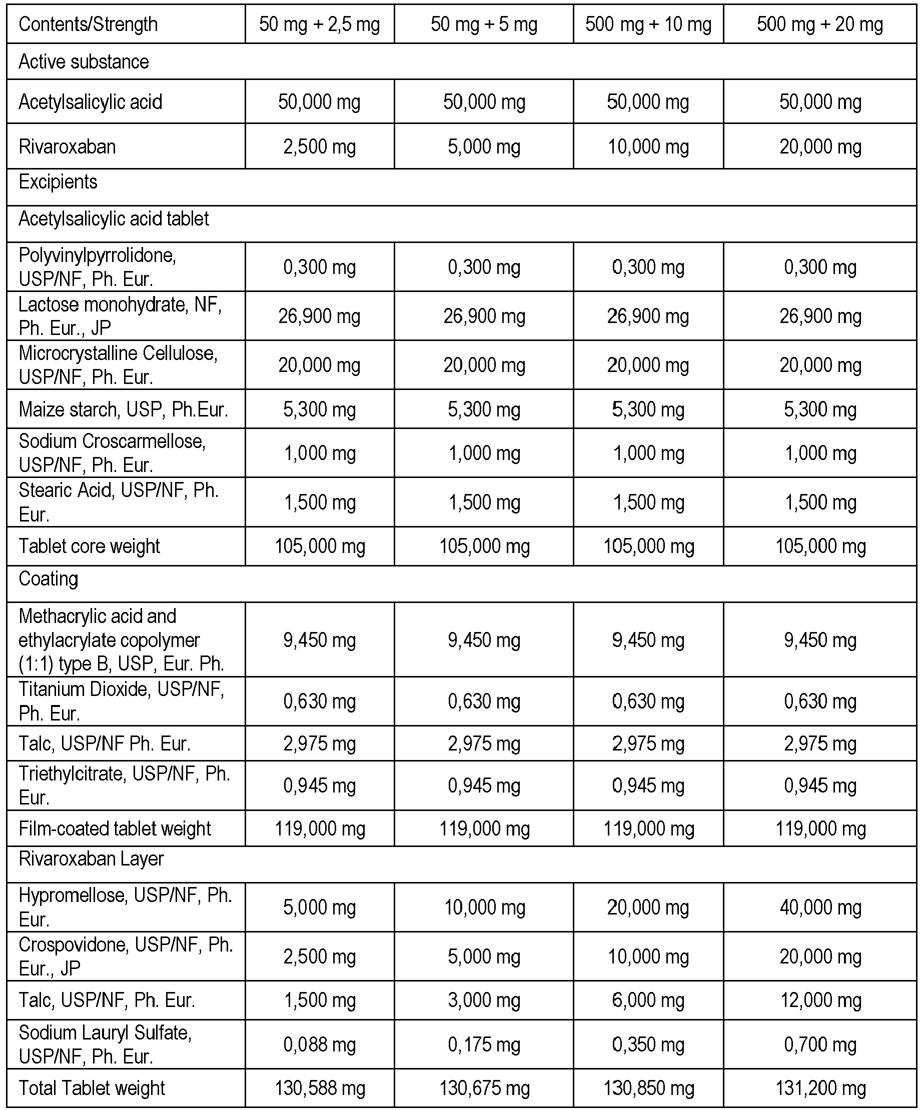

### Manufacturing process (coated tablets)

Preparation by any manufacturing technique applicable for pharmaceutical forms:
- Tablet blend - preferably manufactured by Wet Granulation, Melt Granulation, Twin-Screw Granulation, Dry Granulation or Direct Compression.
- Tablet cores - preferably manufactured by Tableting.
- Enteric Coated tablets - preferably manufactured by Coating.
- API layered enteric Coated tablets - preferably manufactured by Coating. Following example is a manufacturing procedure consisting of: Direct Compression, Tableting, Enteric Coating and API Layering.

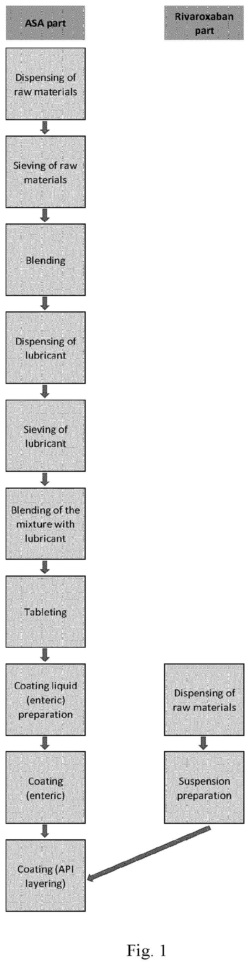

Figure 1 presents manufacturing flowchart of the process described below.

### I. Manufacturing of ASA tablet core

1) Dispensing of raw materials: API and excipients.
2) Sieving of raw materials: API and excipients (without a lubricant), preferably through a sieve of a size of 0.8 mm.
3) Blending, preferably in a suitable tumble mixer, preferably with 85-540 revolutions.
4) Sieving of a lubricant, preferably through a sieve of a size of 0.5 mm.
5) Blending of the mixture with lubricant preferably in a suitable tumble mixer, preferably with 34-90 revolutions.
6) Tableting for a preferable hardness of 40 - 100 N, disintegration time ≤15 min and friability ≤1%.

### II. Manufacturing of ASA enteric coated tablet

1) Coating liquid preparation - addition of all dry components to a liquid vehicle and mix suitably for visually homogenic solution/suspension.
2) Coating of tablet cores manufactured within Step I - with any appropriate tablet coating technique, preferably in a pan coater, with process parameters adjusted to a particular coat grade, machine type and batch size, to a target weight gain.

### III. Manufacturing of Rivaroxaban-layered ASA enteric coated tablet

1) Dispensing of raw materials: API and excipients.
2) Suspension preparation - add all dry components to a liquid vehicle and mix suitably for a homogenic suspension.
3) Coating of tablets manufactured within Step II - with any appropriate tablet coating technique, preferably in a pan coater, to a target weight gain.

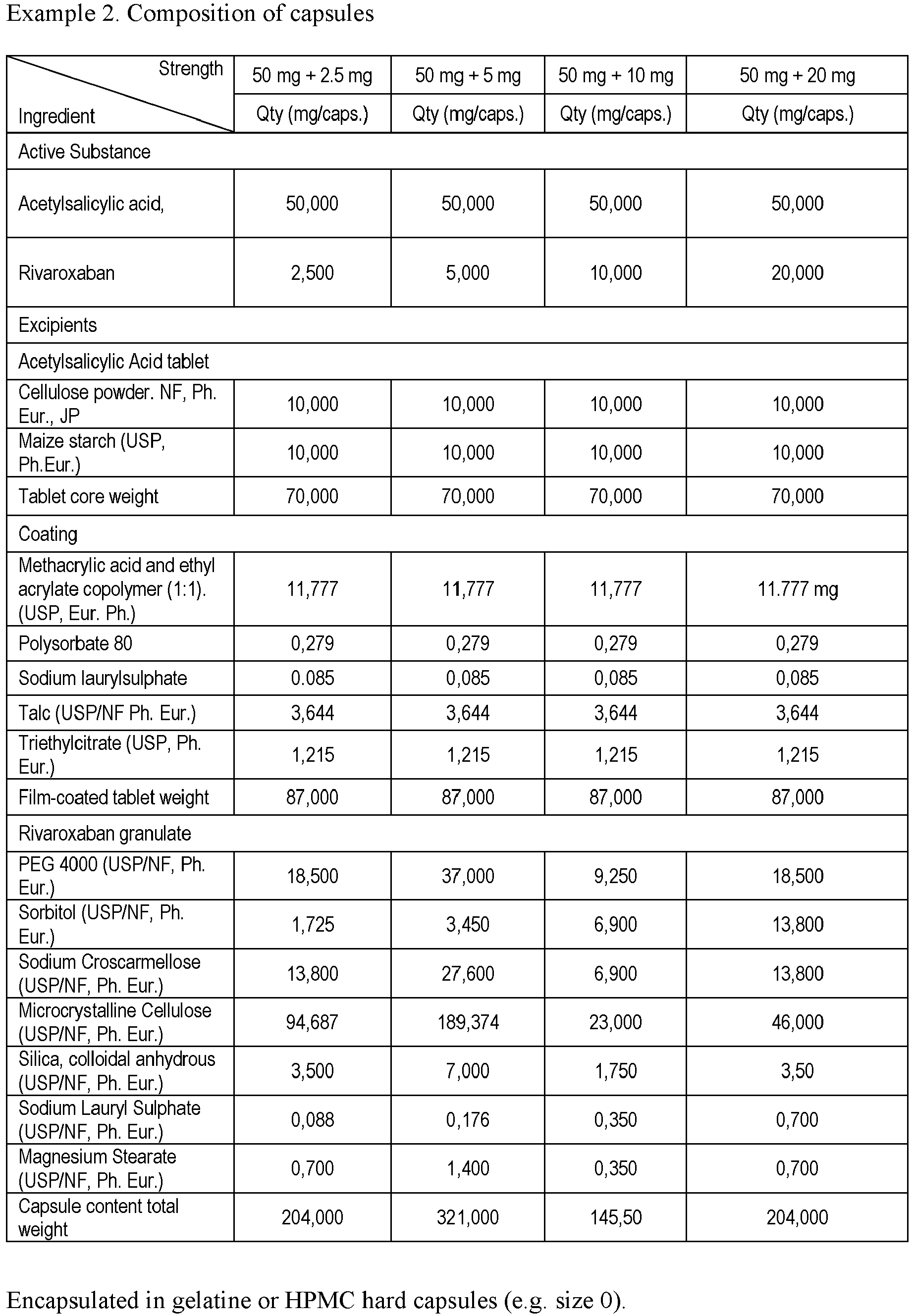

### Manufacturing process (capsules)

Preparation by any manufacturing technique applicable for pharmaceutical forms:
- Tablet blend - preferably manufactured by Wet Granulation, Melt Granulation, Twin-Screw Granulation, Dry Granulation or Direct Compression.
- Tablet cores - preferably manufactured by Tableting.
- Enteric Coated tablets - preferably manufactured by Coating.
- Granulate - preferably manufactured by Wet Granulation, Melt Granulation, Twin-Screw Granulation or Dry Granulation.
- Capsules - preferably manufactured by Encapsulation.
Following example is a manufacturing procedure consisting of: Direct Compression, Tableting, Enteric Coating, Twin-Screw Granulation and Encapsulation.

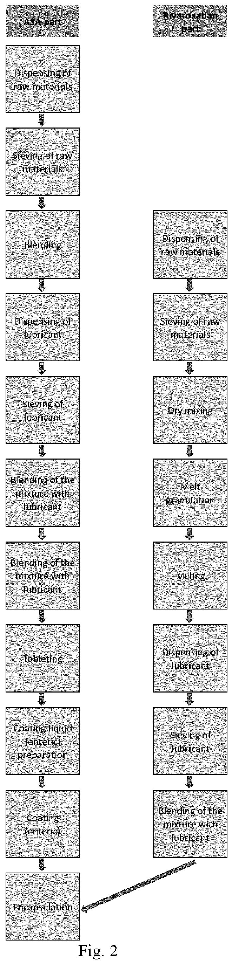

Figure 2 presents manufacturing flowchart of the process described below.

### I. Manufacturing of ASA tablet core

1) Dispensing of raw materials: API and excipients.
2) Sieving of raw materials: API and excipients (without a lubricant), preferably through a sieve of a size of 0.8 mm.
3) Blending, preferably in a suitable tumble mixer, preferably with 85-540 revolutions.
4) Sieving of a lubricant, preferably through a sieve of a size of 0.5 mm.
5) Blending of the mixture with lubricant preferably in a suitable tumble mixer, preferably with 34-90 revolutions.
6) Tableting for a preferable hardness of 40-100 N, disintegration time ≤15 min and friability ≤1%.

### II. Manufacturing of ASA enteric coated tablet

1) Coating (enteric) liquid preparation - addition of all dry components to a liquid vehicle and mix suitably for visually homogenic solution/suspension.
2) Coating (enteric) of tablet cores manufactured within Step I - with any appropriate tablet coating technique, preferably in a pan coater, with process parameters adjusted to a particular coat grade, machine type and batch size, to a target weight gain.

### III. Manufacturing of Rivaroxaban granulate

1. Dispensing of raw materials: API and excipients.
2. Sieving of raw materials: API and excipients, preferably through a sieve of a size of 0.8 mm.
3. Dry mixing of intra-granular phase ingredients, preferably in a suitable tumble mixer, preferably with 85-540 revolutions.
4. Melt granulation of the prepared dry mix in a suitable extruder, preferably at product temperature in range of 40-50°C.
5. Milling of the granulate, preferably with a suitable conical mill or oscillatory bar mill.
6. Sieving of a lubricant, preferably through a sieve of a size of 0.5 mm.
7. Blending the mixture with lubricant preferably in a suitable tumble mixer, preferably with 34-90 revolutions.

### IV. Encapsulation

Encapsulation with a suitable capsule filling machine, technically capable to fill two types of material (tablet and granulate) into one hard capsule shell.

Example 3. Testing methodology and results.

Tablets and capsules described in Examples 1-2 have been tested as below.

### I. Testing of gastric resistance of tablets and capsules (relevant to Aspirin only)

1) Methodology: according to methods given in the respective product monographs (USP monograph for Aspirin delayed-release tablets/capsules, Acid stage)
   Apparatus: basket (Apparatus 1)
   Basket speed: 100 rpm
   Temperature: 37°C
   Medium: 0.1 M hydrochloric acid
   Medium volume: 1000 ml
   Time: 2 h
   Quantitation method: UV/Vis spectrophotometry
   Analytical wavelength: isosbestic point at ca. 280 nm
2) Results: amount of aspirin dissolved - not more than 10% of the label claim

### II. Testing of Aspirin - dissolution at pH 6.8

1) Methodology: according to methods given in the respective product monographs (USP monograph for aspirin delayed-release tablets/capsule, Buffer stage following Acid stage described in point I)
   Apparatus: basket (Apparatus 1)
   Basket speed: 100 rpm
   Temperature: 37°C
   Medium: phosphate buffer pH = 6.8
   Medium volume: 1000 ml
   Time: 90 min
   Quantitation method: UV/Vis spectrophotometry
   Analytical wavelength: isosbestic point at 265 nm
2) Results: amount of aspirin dissolved - not less than Q + 5% of the label claim (Q = 75%)

### III. Testing of Rivaroxaban - dissolution at pH 4.5

1) Methodology: according to methods given in the respective product monograph (Ph. Eur. monograph for Rivaroxaban tablets)
   Apparatus: paddle (Apparatus 2)
   Paddle speed: 75 rpm
   Temperature: 37°C
   Medium: acetate buffer pH = 4.5
   Medium volume: 900 ml
   Time: 30 min
   Quantitation method: HPLC
   Column: with end-capped octadecylsilyl silica gel (3 µm), 60 x 4.0 mm
   Mobile phase: water + acetonitrile (6:4)
   Mobile phase flow rate: 1 ml/min
   Column temperature 40°C
   Injection volume: 10 µl
2) Results: amount of Rivaroxaban dissolved - not less than Q + 5% of the label claim (Q = 80%)

## Claims

1. A pharmaceutical single unit dosage form **characterised in that** it comprises Acetylsalicylic Acid and Rivaroxaban, wherein said single unit dosage form:
- maintains gastric resistance effect of Acetylsalicylic Acid at pH 1.2: < 10% after 2h and simultaneously provides its dissolution at pH 6.8: > 75% within 90 minutes, and
- provides dissolution of Rivaroxaban at pH 4.5 in QC test > 80% within 30 minutes.

2. A pharmaceutical single unit dosage according to claim 1, in form of coated tablet, comprising:
(i) a core comprising: Acetylsalicylic Acid, cellulose and starch, possibly in dry weight ratio 5:1:1, respectively,
(ii) an inner coating comprising: methacrylic acid and ethylacrylate copolymer, Polysorbate 80, sodium laurylsulphate, talc and triethylcitrate, possibly in dry weight ratio 11.777:0.279:0.085:3.644:1.215, respectively,
(iii) an outer coating comprising: Rivaroxaban, HPMC and crospovidone, possibly in dry weight ratio 1:2:1, respectively, and optionally pharmaceutical acceptable excipient, possibly talc.

3. A pharmaceutical single unit dosage according to claim 1, in form of capsule, comprising:
- film coated granules comprising of:
(i) a core comprising of: acetylsalicylic acid, cellulose and starch, possibly in dry weight ratio 5:1:1, respectively,
(ii) a coating comprising of: methacrylic acid and ethyl acrylate copolymer, Polysorbate 80, sodium lauryl sulphate, talc and triethyl citrate, possibly in dry weight ratio 11.777:0.279:0.08:3.644:1.215, respectively,
- dry mixture comprising: rivaroxaban formulated with pharmaceutically acceptable excipients, preferably mixture of: PEG, sorbitol, sodium croscarmellose, cellulose, silica, sodium lauryl sulphate and magnesium stearate, possibly in dry weight ratio from 2,5:9,25:1,725:6,9:23,0:1,75:0,088:0,35 to 20:37,0:13,8:27,6:189,374:7,0:0,7, respectively,
- hard capsule, possibly comprising gelatine or HPMC.

4. A pharmaceutical single unit dosage form according to any one of claims 1 to 3 for use in prevention or treatment of thromboembolic disorders, atherosclerotic thrombotic events, especially in adult patients after acute coronary syndrome (ACS) with elevated cardiac biomarkers, or other patients with chronic diseases.

5. A pharmaceutical single unit dosage form for use according to claim 4 wherein it is dosed in an 12-hour interval.
